# EUROPÄISCHE PATENTSCHRIFT

(11) **EP 3 620 182 B1**
(45) Veröffentlichungstag und Bekanntmachung des Hinweises auf die Patenterteilung: **13.11.2024**
(21) Anmeldenummer: 19193772.1
(22) Anmeldetag: 27.08.2019
(51) Int. Cl.: A61L 9/12, F24C 15/20

(54) **DUNSTABZUGSHAUBE MIT BEDUFTUNGSEINHEIT UMFASSEND EINEN DUFTSTOFFQUELLENHALTER**
EXTRACTION HOOD WITH A FRAGRANCE UNIT CONTAINING A FRAGRANCE SOURCE HOLDER
HOTTE ASPIRANTE AVEC UNE UNITÉ DE DIFFUSION DE PARFUM COMPRENANT UN SUPPORT POUR PARFUM

(30) Priorität: 04.09.2018 DE 102018121527
(43) Veröffentlichungstag der Anmeldung: 11.03.2020
(73) Patentinhaber: Miele & Cie. KG, 33332 Gütersloh (DE)
(72) Erfinder: Karsten, Olaf, 59494 Soest (DE); Hüster, Ingo, 59759 Arnsberg (DE); Wiens, Rudolf, 59494 Soest (DE)

(56) Entgegenhaltungen:
- DE-A1- 102017 100 415
- GB-A- 2 507 635
- US-A1- 2012 091 221
- US-A1- 2014 271 376

## Beschreibung

Die Erfindung betrifft ein Haushaltsgerät gemäß des Patentanspruchs 1 Beispielsweise im Haushalt ist es seit Längerem bekannt, durch zusätzliche Maßnahmen die Raumluft zu verbessern. Als einfachste Maßnahme können frische Blumen aufgestellt werden, welche ihren Duft an die Umgebungsluft abgeben und so einen Raum mit einem angenehmen Geruch versehen können. Hierdurch kann nicht nur der eigene Duft der Blumen im Raum verbreitet sondern es können auch andere Düfte überdeckt werden, welche von Personen als unangenehm empfunden werden könnten. Zu diesem Zweck können beispielsweise auch parfümierte getrocknete Blumen sowie Stoffsäckchen, welche z.B. mit getrocknetem Lavendel gefüllt sind, verwendet werden.

Um den Duftstoff intensiver an die Umgebungsluft abzugeben sind sogenannte Dochtdiffusoren bekannt. Dabei wird ein flexibler oder starrer Festkörper mit einer Seite in einem Behälter mit flüssigem Duftstoff angeordnet. Durch kapillare Effekte an oder in dem Festkörper gelangt die Flüssigkeit aus dem zu einer großen Oberfläche, wo diese dann verdunsten kann und so den Duftstoff an die Raumluft abgibt. Derartige Vorrichtungen sind beispielsweise in der Druckschrift US 2012 0091221 A1 und in der Druckschrift GB 2507635 A offenbart.

Um den Duftstoff wirkungsvoller an die Umgebungsluft abgeben und damit einen größeren Bereich um die Duftstoffquelle herum mit dem angenehmen Geruch erfüllen zu können, ist es bekannt, künstliche Duftstoffquellen zu verwenden, welche aktiv einen Luftstrom erzeugen können. Derartige Beduftungsvorrichtungen, welche auch als Bedufter bezeichnet werden können, weisen eine Beduftungseinheit auf, welche einen Duftstoff aufnehmen, speichern und an die Umgebung aktiv abgeben kann. Üblicherweise wird ein austauschbarer Duftstoffbehälter, auch Duftstoffkapsel genannt, als Duftstoffquelle verwendet, welcher eine Duftstoffflüssigkeit aufweist. Ein Beispiel für eine derartige aktive Vorrichtung ist in der Druckschrift US 2014 0271376 A1 gezeigt, wobei eine Portion eines flüssigen Duftstoffes aus einem unter Druck stehendem Behälter durch öffnen eines Ventiles entnommen werden kann und dann mittels einer Düse im Raum verteilt wird.

Es kann ein Luftstrom von Umgebungsluft an der Duftstoffquelle vorbeigeführt werden, um den Duftstoff mit sich mit zu führen und an die Umgebung der Beduftungsvorrichtung abzugeben. Der Luftstrom durch die Beduftungseinheit hindurch kann durch einen Lüfter der Beduftungseinheit erfolgen, welcher Umgebungsluft durch eine Einlassöffnung ansaugen und durch eine Auslassöffnung wieder an die Umgebung abgeben kann. Derartige Beduftungsvorrichtungen sind als elektrische Raumbedufter beispielsweise von der Fa. Gries Deco Company GmbH unter dem Markenname "Air Pearls" bekannt.

Auch ist es bekannt, eine derartige Beduftungseinheit in ein Haushaltsgerät in Form eines Wäschetrockners oder eines Waschtrockners zu integrieren, um, wie zuvor beschrieben, Duftstoff an die Prozessluft abzugeben, welche der Wäsche zugeführt wird um diese zu trocknen. Auf diese Art und Weise kann neben der Trocknung der Wäsche deren Parfümierung im selben Arbeitsschritt erreicht werden.

Aus der Druckschrift DE 102017100415 A1 ist eine Dunstabzugshaube mit einer Beduftung bekannt.

Üblicherweise ist in derartigen Haushaltsgeräten ein Duftstoffquellenhalter vorgesehen, welcher die Duftstoffquelle aufnehmen und halten kann, so dass ein Luftstrom an der Duftstoffquelle vorbeigeführt werden kann, um den Duftstoff aufzunehmen und mit sich mit zu führen.

Nachteilig ist bei bekannten Beduftungseinheiten bzw. deren Duftstoffquellenhaltern, dass falls die Duftstoffflüssigkeit ausläuft, diese in die Beduftungseinheit eindringen kann. Hierdurch kann die ausgelaufene Duftstoffflüssigkeit in Bereiche der Beduftungseinheit bzw. der Beduftungsvorrichtung wie z.B. des Haushaltsgeräts gelangen, welche für den Benutzer nur schwierig bis gar nicht zu erreichen und zu reinigen sein können.

Der Erfindung stellt sich somit das Problem, einen Duftstoffquellenhalter der eingangs beschriebenen Art bereitzustellen, welcher verhindern kann, dass ausgelaufene Duftstoffflüssigkeit in die Beduftungseinheit und bzw. oder die Beduftungsvorrichtung wie z.B. in das Haushaltsgerät gelangen kann. Dies soll möglichst zuverlässig, einfach und bzw. oder kostengünstig ermöglicht werden. Zumindest soll eine Alternative zu bekannten Duftstoffquellenhaltern geschaffen werden.

Erfindungsgemäß wird dieses Problem durch ein Haushaltsgerät mit den Merkmalen des Patentanspruchs 1 gelöst. Vorteilhafte Ausgestaltungen und Weiterbildungen der Erfindung ergeben sich aus den nachfolgenden abhängigen Ansprüchen.

Somit betrifft die vorliegende Erfindung ein Haushaltsgerät, welches als eine Dunstabzugshaube ausgeführt ist, mit einer Beduftungseinheit mit wenigstens einer Aufnahme für wenigstens eine Duftstoffquelle.

Der vorliegenden Erfindung liegt dabei die Erkenntnis zugrunde, dass Duftstoffe üblicherweise in flüssiger Form verwendet werden. Durch die horizontale Ausrichtung einer aufgenommenen Duftstoffquelle in dem Duftstoffquellenhalter mittels dessen Aufnahme kann somit üblicherweise verhindert werden, dass eine Duftstoffquellenflüssigkeit auslaufen kann, da eine Öffnung einer Duftstoffquelle zur Abgabe des Duftstoffs üblicherweise vertikal in der Höhe nach oben ausgerichtet ist.

Gemäß der vorliegenden Erfindung weist der Duftstoffquellenhalter wenigstens ein Auffangelement auf, welches in der Höhe unterhalb der Aufnahme angeordnet ist. Das Aufnahmeelement kann auch als Auffangbehälter oder als Auffangwanne bezeichnet werden. Auf diese Art und Weise kann erreicht werden, dass für den Fall eines Austritts eines flüssigen Duftstoffs dieser durch das Auffangelement aufgefangen werden kann. Hierdurch kann verhindert werden, dass die Flüssigkeit in eine Beduftungseinheit bzw. in eine Beduftungsvorrichtung, wie zum Beispiel in ein Haushaltsgerät, eindringen kann. Hierdurch könnte die Flüssigkeit in Bereiche gelangen, welche für einen Benutzer gar nicht oder lediglich schwierig erreichbar sind und damit gar nicht oder lediglich schwierig von der Duftstoffflüssigkeit gereinigt werden können.

Gemäß einem weiteren Aspekt der vorliegenden Erfindung erstreckt sich das Auffangelement ausreichend gegenüber einer in der Aufnahme aufgenommenen Duftstoffquelle, um aus der Duftstoffquelle austretende Duftstoffflüssigkeit vollständig aufzufangen. In anderen Worten ist das Auffangelement in der Horizontalen sich ausreichend erstreckend ausgebildet, um die in der Aufnahme des Duftstoffquellenhalters aufgenommene Duftstoffquelle ausreichend zu überragen, so dass heruntertropfende Flüssigkeit in allen Richtungen aufgefangen werden kann. Hierdurch kann erreicht werden, dass auslaufende Flüssigkeit aus der Duftstoffquelle von dem Auffangelement in jedem Fall sicher aufgefangen werden kann. Anders ausgedrückt kann auf diese Art und Weise verhindert werden, dass austretende Duftstoffflüssigkeit aus der Duftstoffquelle an dem Auffangelement vorbei in die Beduftungseinheit gelangen kann. Gemäß einem weiteren Aspekt der vorliegenden Erfindung ist das Auffangelement ausreichend groß ausgebildet, um die gesamte aus der Duftstoffquelle austretende Duftstoffflüssigkeit aufzunehmen. Mit anderen Worten ist das Volumen bzw. das Fassungsvermögen des Auffangelements ausreichend groß ausgebildet, damit der gesamte Inhalt einer einzelnen Duftstoffquelle in dem Auffangelement aufgenommen werden kann. Hierdurch kann verhindert werden, dass das Auffangelement durch die aufgenommene Duftstoffflüssigkeit überlaufen kann.

Gemäß der vorliegenden Erfindung ist der Duftstoffquellenhalter ausgebildet, aus der Beduftungseinheit entnommen und in die Beduftungseinheit eingeführt zu werden. Hierdurch kann der Duftstoffquellenhalter entnommen werden, um vom Benutzer mit einer frischen Duftstoffquelle versehen zu werden. In diesem Zustand kann der Duftstoffquellenhalter wieder in die Beduftungseinheit eingeführt werden, um verwendet zu werden. Dabei kann auch beim Entnehmen bzw. beim Einführen des Duftstoffquellenhalters durch die horizontale Ausrichtung der Aufnahme ein Auslaufen von Duftstoffflüssigkeit aus der Duftstoffquelle vermieden werden.

Gemäß der vorliegenden Erfindung weist der Duftstoffquellenhalter wenigstens ein Abstellelement auf, welches ausgebildet ist, dem entnommenen Duftstoffquellenhalter einen sicheren Stand zu bieten, in welchem die Aufnahme horizontal ausgerichtet ist. Hierdurch kann auch im entnommenen Zustand des Duftstoffquellenhalters, wenn dieser zum Beispiel auf einer Oberfläche zum Reinigen oder zum Austausch der Duftstoffquelle abgestellt wird, mittels des Abstellelements sichergestellt werden, dass die horizontale Ausrichtung der Aufnahme des Duftstoffquellenhalters gewahrt bleibt. Gleichzeitig kann ein sicherer Stand des Duftstoffquellenhalters ermöglicht werden, so dass dieser nicht kippen kann. Hierdurch kann jeweils verhindert werden, dass eine aufgenommene Duftstoffquelle auslaufen kann.

Gemäß einem weiteren Aspekt der vorliegenden Erfindung erstreckt sich das Abstellelement über die gesamte horizontale Erstreckung des Duftstoffquellenhalters. Mit anderen Worten ist das Abstellelement vollflächig ausgebildet und erstreckt sich über die gesamte Breite und Tiefe des Duftstoffquellenhalters. Die Abstellfläche des Abstellelements kann dabei eine beliebige Geometrie aufweisen, vorzugsweise jedoch rechteckig ausgebildet sein, was üblicherweise einen sicheren Stand gewährleisten kann. Hierdurch kann eine möglichst große Standfläche des Abstellelements erreicht werden, um einen möglichst sicheren Stand zu gewährleisten.

Gemäß einem weiteren Aspekt der vorliegenden Erfindung ist das Abstellelement als mehrere Standfüße ausgebildet. Auf diese Art und Weise können über die Standfüße mehrere Kontaktpunkte zu einem Untergrund ausgebildet werden, was einem Kippen des Duftstoffquellenhalters entgegenwirken kann. Auch können Unebenheiten eines Untergrunds durch die Kontaktaufnahme über mehrere Standfüße besser vermieden werden als durch ein vollflächiges Abstellelement. Andererseits kann die Ausbildung von mehreren Standfüßen aufwendiger als die Ausbildung eines vollflächigen Abstellelements sein.

Gemäß einem weiteren Aspekt der vorliegenden Erfindung ist das Abstellelement als vier Standfüße ausgebildet, welche vorzugsweise in den Ecken des Duftstoffquellenhalters angeordnet sind. Hierdurch kann eine sichere Gewichtsverteilung des Duftstoffquellenhalters über die vier Standfüße auf einen Untergrund erfolgen, so dass einem Kippen sicher entgegengewirkt werden kann. Hierbei die vier Standfüße vorzugsweise auf die Ecken des Duftstoffquellenhalters zu verteilen, kann bei einem Duftstoffquellenhalter mit einer rechteckigen bzw. quadratischen Grundfläche für einen besonders sicheren Stand sorgen.

Gemäß einem weiteren Aspekt der vorliegenden Erfindung ist zwischen den Standfüßen wenigstens ein Verbindungselement, vorzugsweise ein Rastelement, besonders vorzugsweise ein Rasthaken, für eine Halterungseinheit, vorzugsweise für einen Push-Push-Verriegelungsmechanismus, der Beduftungseinheit angeordnet, wobei das Verriegelungselement in der Höhe gegenüber den Standfüßen gleich lang oder zurückversetzt ausgebildet ist. Mittels eines derartigen Verriegelungselements kann der Duftstoffquellenhalter, welcher entnehmbar hinsichtlich der Belüftungseinheit ausgebildet ist, in dieser gehalten werden.

Hierbei ein Rastelement und vorzugsweise einen Rasthaken zu verwenden, kann eine einfachere und sichere Verbindung ermöglichen. Seitens der Beduftungseinheit einen Push-Push-Verriegelungsmechanismus zu verwenden, kann vorteilhaft sein, weil der Duftstoffquellenhalter durch ein erstes Drücken des Benutzers in der Höhe von oben in dem Push-Push-Verriegelungsmechanismus gehalten und durch ein erneutes Drücken aus diesem nach oben gelöst werden kann. Gleichzeitig kann durch dieses Lösen der Duftstoffquellenhalter in der Höhe dem Benutzer zugeführt werden, so dass dieser vom Benutzer einfacher gegriffen und entnommen werden kann.

Dabei das Verriegelungselement zwischen den Standfüßen anzuordnen und dieses nicht über die Standfüße in der Höhe nach unten hervorragen zu lassen, kann sowohl eine platzsparende Anordnung des Verriegelungselements ermöglichen als auch gleichzeitig verhindern, dass die Standfestigkeit, welche durch die Standfüße erreicht wird, durch das Verriegelungselement beeinträchtigt wird.

Gemäß einem weiteren Aspekt der vorliegenden Erfindung ist das Auffangelement als Abstellelement ausgebildet. Auf diese Art und Weise können die zuvor beschriebenen Vorteile und Eigenschaften des Auffangelements und des Abstellelements miteinander kombiniert durch ein einziges Bauteil des Duftstoffquellenhalters werden. Ob als vollflächiges Abstellelement oder als Abstellelement in Form von Standfüßen kann dieses jeweils nach oben hin derart hohl ausgebildet werden, so dass durch das Abstellelement gleichzeitig das Auffangelement ausgebildet werden kann. Dies kann die Komplexität des Duftstoffquellenhalters verringern sowie Material, Kosten, Bauraum und bzw. oder Gewicht sparen. Mit anderen Worten kann eine maximale Funktionalität bei geringem Bauraum ermöglicht werden.

Gemäß einem weiteren Aspekt der vorliegenden Erfindung ist der Duftstoffquellenhalter wenigstens zweiteilig ausgebildet und weist wenigstens ein Oberteil und wenigstens ein Unterteil auf, wobei das Oberteil wenigstens die Aufnahme aufweist und wobei das Unterteil wenigstens ein Auffangelement und bzw. oder wenigstens ein Abstellelement aufweist. Auf diese Art und Weise können das Oberteil und das Unterteil jeweils einstückig zum Beispiel als Spritzgussteil hergestellt und anschließend zu dem Duftstoffquellenhalter verbunden werden. Dies kann eine einfache und kostengünstige Konstruktion, Herstellung sowie Montage des Duftstoffquellenhalters ermöglichen.

Gemäß einem weiteren Aspekt der vorliegenden Erfindung weist das Oberteil wenigstens ein Verbindungselement, vorzugsweise eine Mehrzahl von Verbindungselementen, und das Unterteil wenigstens eine Verbindungselementaufnahme, vorzugsweise eine Mehrzahl von Verbindungselementaufnahmen, auf, oder umgekehrt, wobei vorzugsweise das Verbindungselement als Rasthaken und die Verbindungselementaufnahme als Rasthakenaufnahme ausgebildet ist. Hierdurch kann es ermöglicht werden, dass das Oberteil und das Unterteil miteinander verbunden werden. Dies über wenigstens einen Rasthaken und eine korrespondierende Rasthakenaufnahme vorzusehen, kann einen einfachen, aber sicheren formschlüssigen und bzw. oder kraftschlüssigen Halt ermöglichen. Auch können ein Rasthaken und eine Rasthakenaufnahme an Spritzgussteilen einstückig sehr einfach ausgebildet werden.

Als besonderer Vorteil der Ausgestaltung eines elektrischen Haushaltsgerätes mit einer Beduftungseinheit liegt darin, dass die mit einem Lüfter zur Erzeugung eines Luftstroms ausgestattete Beduftungseinheit keine eigene elektrische Versorgung benötigt, sondern sich der elektrischen Versorgung des Haushaltsgerätes bedient.

Auch wird durch die Integration in ein Haushaltsgerät vermieden, dass ein weiteres, ggf. mobiles Gerät zusätzlichen Raum in der Wohnung belegt.

Als besonderer Vorteil der Ausgestaltung einer Dunstabzugshaube mit einer Beduftungseinheit liegt darin, dass der Duft gezielt an einem Ort ausgetragen werden kann, an dem als negativ empfundene Gerüche konzentriert anfallen. So ist eine effektive Überdeckung der als negativ empfundene Gerüche mit einem hohen Wirkungsgrad möglich. Auch für die alternative Belüftung eines Raumes, unabhängig von einem Kochbetrieb ist die Dunstabzugshaube ein günstiger Ort. Eine Dunstabzugshaube ist, beispielsweise über einem Kochfeld, insbesondere frei hängend über einer Kochinsel, strömungsgünstig im Raum angeordnet und kann somit den Duft wirkungsvoll verteilen.

Ein Ausführungsbeispiel der Erfindung ist in den Zeichnungen rein schematisch dargestellt und wird nachfolgend näher beschrieben. Es zeigt
- Figur 1: eine Explosionszeichnung eines Duftstoffquellenhalters;
- Figur 2: den Duftstoffquellenhalter perspektivisch von einer Seite;
- Figur 3: den Duftstoffquellenhalter perspektivisch von der gegenüberliegenden Seite; und
- Figur 4: eine perspektivische Darstellung einer Beduftungsvorrichtung in Form einer Dunstabzugshaube.

Die o.g. Figuren werden in kartesischen Koordinaten betrachtet. Es erstreckt sich eine Längsrichtung X, welche auch als Tiefe X bezeichnet werden kann. Senkrecht zur Längsrichtung X erstreckt sich eine Querrichtung Y, welche auch als Breite Y bezeichnet werden kann. Senkrecht sowohl zur Längsrichtung X als auch zur Querrichtung Y erstreckt sich eine vertikale Richtung Z, welche auch als Höhe Z bezeichnet werden kann.

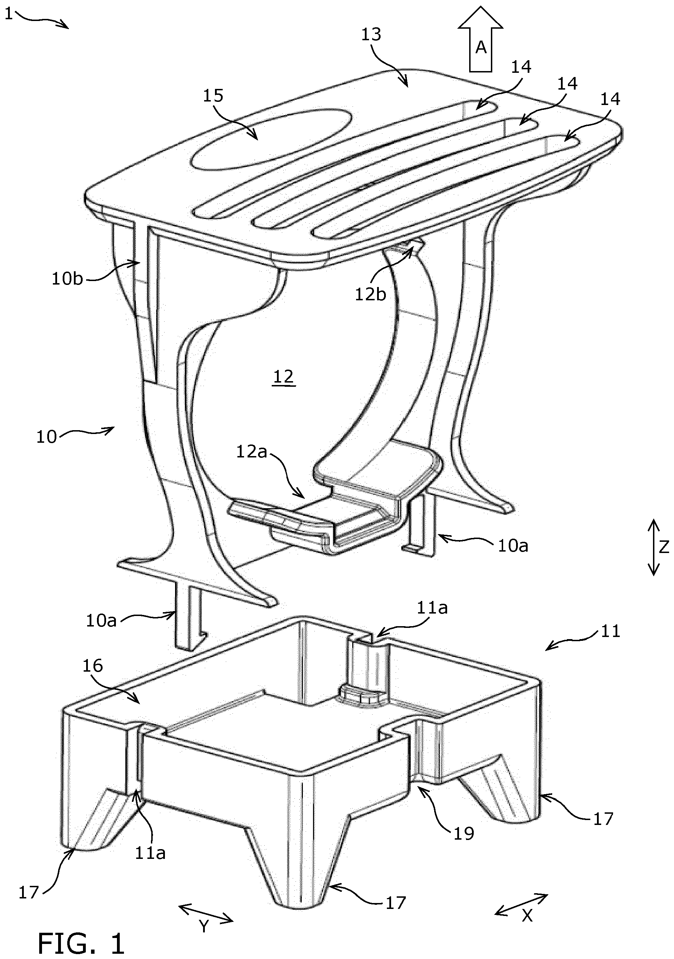

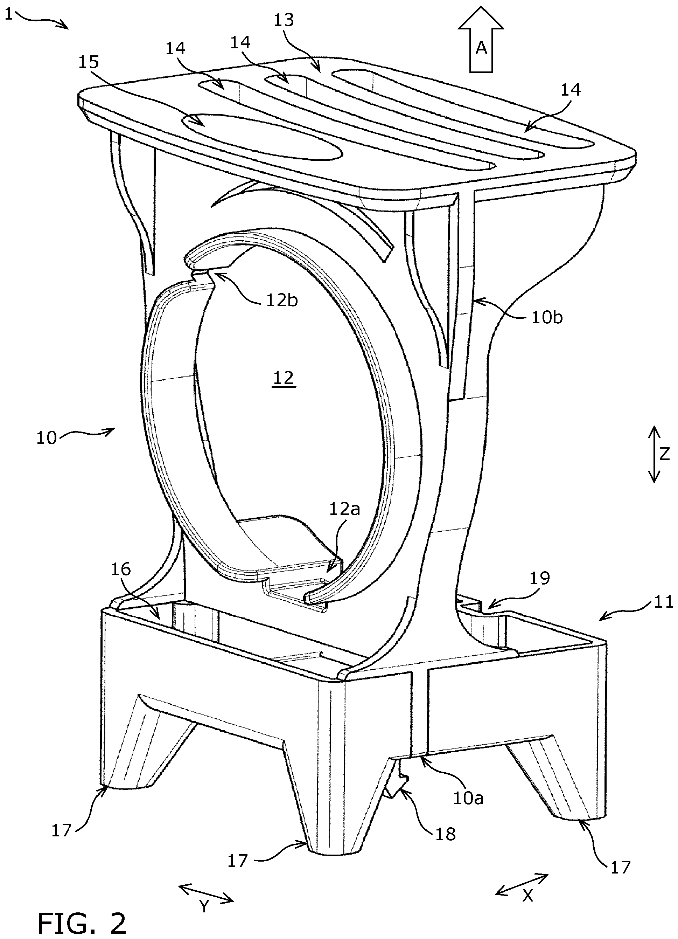

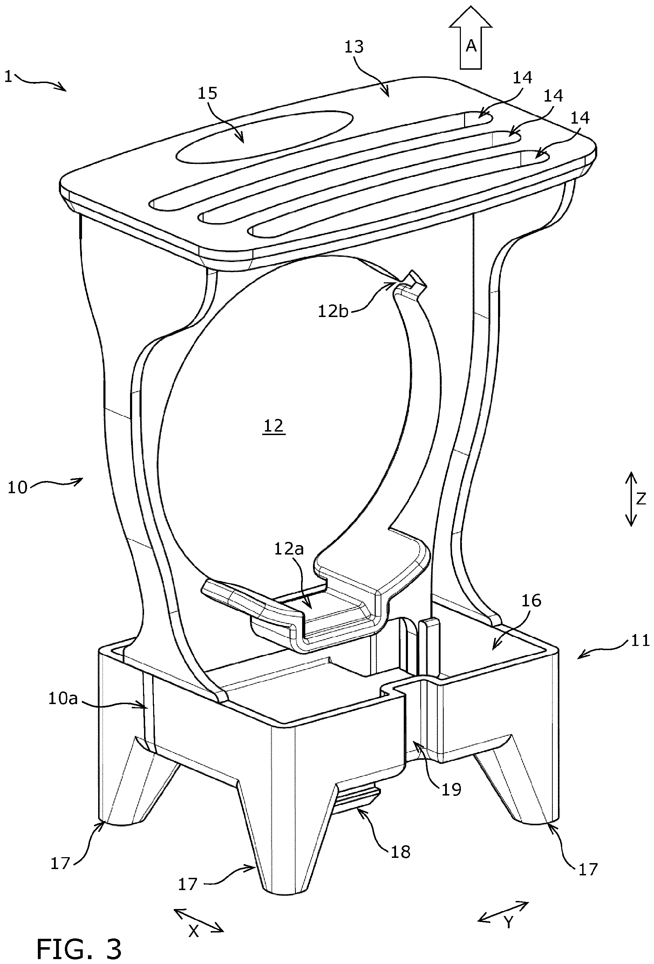

Fig. 1 zeigt eine Explosionszeichnung eines Duftstoffquellenhalters 1. Fig. 2 zeigt den Duftstoffquellenhalter 1 perspektivisch von einer Seite. Fig. 3 zeigt den Duftstoffquellenhalter 1 perspektivisch von der gegenüberliegenden Seite.

Der Duftstoffquellenhalter 1 dient der Aufnahme einer Duftstoffquelle (nicht dargestellt). Als Duftstoffquelle können insbesondere eine flüssigkeitsgefüllte Duftstoffkapsel verwendet werden, welche auch als Duftstoffflakon bezeichnet werden kann. Entsprechend kann der Duftstoffquellenhalter 1 auch als Duftstoffbehälterhalter 1, als Duftstoffkapselhalter 1 oder als Duftflakonhalter 1 bezeichnet werden.

Der Duftstoffquellenhalter 1 besteht aus einem Oberteil 10 und aus einem Unterteil 11, welche jeweils separat als Spritzgussteile hergestellt und anschließend miteinander verbunden werden. Das Oberteil 10 weist hierzu zwei Verbindungselemente 10a in Form von Rasthaken 10a auf, welche an der Unterseite des Oberteils 10 sich in der Höhe Z nach unten erstreckend ausgebildet sind. In der Querrichtung Y liegen sich die beiden Rasthaken 10a gegenüber. Das Unterteil 11 weist zwei korrespondierende Verbindungselementaufnahmen 11a in Form von zwei Rasthakenaufnahmen 11a auf, welche sich in der Höhe Z erstreckende Nuten ausgebildet und seitlich in der Querrichtung Y einander gegenüberliegend angeordnet sind. Das Oberteil 10 kann somit durch Aufstecken in der Höhe Z von oben auf das Unterteil 11 mit diesem verbunden werden, indem die Rasthaken 10a in die Rasthakenaufnahmen 11a eingreifen und schließlich verrasten.

Das Oberteil 10 weist auf einer Seite ein Führungselement 10b in Form eines Vorsprungs 10b auf, siehe Fig. 1 und Fig. 2. Das Führungselement 10b kann in eine korrespondierende Aussparung der Beduftungseinheit eingreifen, um beim Einsetzen des Duftstoffquellenhalters 1 in eine entsprechende Aufnahme der Beduftungseinheit geführt zu werden. Dies kann das Einsetzen für den Benutzer erleichtern und insbesondere ein Verkanten verhindern.

Das Oberteil 10 des Duftstoffquellenhalters 1 weist eine Aufnahme 12 der Duftstoffquelle auf. Die Aufnahme 12 ist im Wesentlichen kreisrund ausgebildet und erstreckt sich mit ihrer Mittelachse in der horizontalen Ebene entlang der Längsrichtung X. Auf diese Art und Weise kann die Duftstoffquelle in der Längsrichtung X von einer Seite in die Aufnahme 12 eingeführt werden. Um in dieser Position sicher gehalten zu werden, weist die Aufnahme 12 eine Aufnahme 12a für ein Verbindungselement der Duftstoffquelle auf. Diese Aufnahme 12a kann nicht nur einer Führung der Duftstoffquelle beim Einsetzen dienen, sondern auch durch beispielsweise ein Verrasten einen sicheren Halt des Duftstoffquelle in der Aufnahme 12 der Duftstoffquelle gewährleisten. Ferner weist die Aufnahme 12 der Duftstoffquelle ein Codierungselement 12b für die Duftstoffquelle auf, welches als Aussparung an einer definierten Stelle angeordnet ist. Durch das Codierungselement 12b kann eine Duftstoffquelle lediglich in einer Orientierung eingesetzt werden, so dass eine bestimmungsgemäße Verwendung gewährleistet werden kann.

In der Höhe Z nach oben schließt das Oberteil 10 des Duftstoffquellenhalters 1 mit einer Oberseite 13 ab, welche auch als Außenseite 13 bezeichnet werden kann. In der Oberseite 13 sind mehrere Auslassöffnungen 14 in Form von Auslassschlitzen 14 angeordnet, durch welche hindurch ein Luftstrom A aus dem Inneren des Duftstoffquellenhalters 1 an die Umgebung abgegeben werden kann.

Auch weist die Oberseite 13 ein Betätigungselement 15 in Form einer Fingermulde 15 auf, über welche es dem Benutzer ermöglicht werden kann, den Duftstoffquellenhalter 1 zu ertasten und haptisch zu erkennen. Auch kann über das Drücken des Duftstoffquellenhalters 1 im Bereich der Fingermulde 15 ein Verriegelungsmechanismus der Beduftungseinheit betätigt werden, wie weiter unten noch näher erläutert werden wird.

Das Unterteil 11 des Duftstoffquellenhalters 1 ist dazu ausgebildet, gleich zwei Funktionen auf einmal zu erfüllen. Zum einen ist das Innere des Unterteils 11 als Auffangelement 16 ausgebildet, die Duftstoffflüssigkeit der Duftstoffquelle aufzufangen, falls diese aus der Duftstoffquelle ungewollt austreten sollte. Daher kann das Auffangelement 16 auch als Auffangbehälter 16 oder als Auffangwanne 16 bezeichnet werden.

Das Unterteil 11 ist dabei derartig ausgebildet, dass sich das Auffangelement 16 zum einen in der Längsrichtung X sowie in der Querrichtung Y ausreichend unterhalb der in der Aufnahme 12 aufgenommenen Duftstoffquelle erstreckt, so dass aus der Duftstoffquelle austretende Duftstoffflüssigkeit sicher von allen Seiten vom Auffangelement 16 aufgefangen werden kann. Mit anderen Worten ist das Auffangelement 16 ausreichend in der Horizontalen dimensioniert, um ein Vorbeilaufen der austretenden Duftstoffflüssigkeit an dem Auffangelement 16 zu verhindern. Zum anderen ist das Auffangelement 16 von seinem Volumen her derart ausgebildet, dass der gesamte Inhalt einer vollständig gefüllten Duftstoffquelle auch von dem Auffangelement 16 aufgenommen werden kann. Hierdurch kann sichergestellt werden, dass das Auffangelement 16 nicht überlaufen kann.

Durch diese Eigenschaften des Auffangelements 16 kann ein Duftstoffquellenhalter 1 geschaffen werden, welcher das Austreten von Duftstoffflüssigkeit über den Duftstoffquellenhalter 1 hinaus in die Beduftungseinheit sicher verhindern kann. Hierdurch kann insbesondere verhindert werden, dass ein Benutzer die Beduftungseinheit von ausgelaufener Duftstoffflüssigkeit reinigen muss. Dies ist insbesondere vorteilhaft, falls die Beduftungseinheit Bereiche aufweist, welche für den Benutzer gar nicht oder nur schwierig zu erreichen und entsprechend gar nicht oder schwierig zu reinigen wären.

Das Unterteil 11 des Duftstoffquellenhalters 1 ist ferner dazu ausgebildet, ein Abstellelement 17 in Form von vier Standfüßen 17 aufzuweisen. Die vier Standfüße 17 sind an den Ecken des rechteckig ausgebildeten Unterteils 11 angeordnet. Die vier Standfüße 17 sind hohl ausgebildet, um das Volumen des Auffangelements 16 zu vergrößern. Mittels der vier Standfüße 17 kann der Duftstoffquellenhalter 1, wenn er aus der Beduftungseinheit entnommen ist, sicher auf einem ebenen Untergrund abgestellt werden. Durch den sicheren Stand des entnommenen Duftstoffquellenhalters 1 kann ebenfalls verhindert werden, dass die Duftstoffflüssigkeit aus der Duftstoffquelle auslaufen kann. Dies begünstigt insbesondere die horizontale Ausrichtung der Aufnahme 12 des Duftstoffquellenhalters 1.

Mittig zwischen den vier Standfüßen 17 des Unterteils 11 des Duftstoffquellenhalters 1 ist ein Verbindungselement 18 für eine Halterungseinheit der Beduftungseinheit vorgesehen. Mittels dieses Verbindungselements 18 kann der Duftstoffquellenhalter 1 in der Beduftungseinheit aufgenommen und gehalten werden. Hierzu vorzugsweise das Verbindungselement 18 als Rastelement 18 bzw. als Rasthaken 18 auszubilden, kann es begünstigen, seitens der Beduftungseinheit einen Push-Push-Verriegelungsmechanismus zu verwenden. Mittels eines derartigen Verriegelungsmechanismus kann der Duftstoffquellenhalter 1 in der Beduftungseinheit zum einen gehalten und zum anderen auf Drücken durch den Benutzer über die Fingermulde 15 der Oberseite 13 des Oberteils 10 des Duftstoffquellenhalters 1 in der Höhe Z nach oben ausgegeben werden. Dies kann es dem Benutzer erleichtern, den Duftstoffquellenhalter 1 mittels seiner Finger zu ergreifen. Dies kann auch die Entnahme für den Benutzer erleichtern. Auch dies kann dazu beitragen, dass beim Entnehmen bzw. auch beim Einführen der Duftstoffquelle aus dieser keine Duftstoffflüssigkeit austreten kann.

Auf einer seiner seitlichen Kanten weist das Unterteil 11 des Duftstoffquellenhalters 1 eine Aufnahme 19 für ein Führungselement der Beduftungseinheit auf. Diese Aufnahme 19 ist vorzugsweise als Nut 19 für einen Führungszapfen ausgebildet. Hierdurch kann eine Führung des Unterteils 11 des Duftstoffquellenhalters 1 beim Einsetzen in eine entsprechende Aufnahme der Beduftungseinheit erfolgen. Auch dies kann ein Auslaufen von Duftstoffflüssigkeit verhindern sowie es dem Benutzer erleichtern, den Duftstoffquellenhalter 2 in die Beduftungseinheit einzuführen.

kann somit ein derartiger Duftstoffquellenhalter 1 von einem Benutzer aus einer Beduftungseinheit durch Betätigen des Push-Push-Verriegelungsmechanismus der Beduftungseinheit über die Fingermulde 15 der Oberseite 13 des Oberteils 10 des Duftstoffquellenhalters 1 erfolgen. Der aus der Beduftungseinheit nach oben in der Höhe Z hervorragende Duftstoffquellenhalter 1 kann von dem Benutzer mit den Fingern gegriffen und nach oben hinausgezogen werden. Anschließend kann der entnommene Duftstoffquellenhalter 1 auf einem Untergrund vom Benutzer abgestellt werden. Hierbei kann ein sicherer Stand und Halt ohne Verkippen über die vier Standfüße 17 ermöglicht werden.

In dieser Lage kann von dem Benutzer eine frische, das heißt vollständig gefüllte, Duftstoffquelle in die entsprechende Aufnahme 12 des Oberteils 10 des Duftstoffquellenhalters 1 eingeführt werden. Diese kann dort an der entsprechenden Aufnahme 12a verrastet werden. Durch die horizontale Ausrichtung der Aufnahme 12 kann die Duftstoffflüssigkeit nicht aus der Duftstoffquelle austreten. Gleichzeitig kann durch den sicheren Stand des Duftstoffquellenhalters 1, welcher über die vier Standfüße 17 erreicht wird, während dieses Vorgangs ein Umkippen und damit Auslaufen der Duftstoffflüssigkeit verhindert oder zumindest erschwert werden.

In diesem Zustand kann der Duftstoffquellenhalter 1 vom Benutzer mit den Fingern gegriffen und angehoben werden. Der Benutzer kann den Duftstoffquellenhalter 1 dann in dieser vertikalen Ausrichtung in die entsprechende Aufnahme der Beduftungseinheit in der Höhe Z von oben einführen, was zum einen über den Führungszapfen der Beduftungseinheit und die korrespondierende Nut 19 des Unterteils 11 des Duftstoffquellenhalters 1 vereinfacht werden kann. Gleichzeitig kann hierdurch eine Codierung des Duftstoffquellenhalters 1 erfolgen, so dass dieser lediglich in einer einzigen Orientierung in die Aufnahme der Beduftungseinheit eingeführt werden kann.

Während dieses Einführens kann das Führungselement 10b des Oberteils 10 des Duftstoffquellenhalters 1 den Abschluss dieser Bewegung unterstützen. Durch das Drücken auf die Fingermulde 15 der Oberseite 13 des Oberteils 10 des Duftstoffquellenhalters 1 seitens des Benutzers kann der Duftstoffquellenhalter 1 sicher in der Beduftungseinheit verrastet werden.

Auf diese Art und Weise kann ein Auslaufen von Duftstoffflüssigkeit sowohl während des Einsetzens der Duftstoffquelle in die Aufnahme 12 des Duftstoffquellenhalters 1 als auch darüber hinaus beim Einführen des Duftstoffquellenhalters 1 in die Beduftungseinheit verhindert werden.

Die Beduftungsvorrichtung kann gemäß einer nicht dargestellten Ausführungsform in Form einer Dunstabzugshaube ausgebildet sein. Die Dunstabzugshaube weist eine Deckenhalterung auf, welche ausgebildet ist, in der Höhe Z von unten an einer Decke einer Küche oberhalb eines Kochfeldes montiert zu werden. An der Unterseite der Deckenhalterung sind vier Haltemittel in Form von vier Seilen angeordnet, welche sich in der Höhe Z nach unten erstrecken.

Die eigentliche Dunstabzugshaube weist einen unteren Gehäusemantel sowie einen oberen Gehäusemantel auf. Der untere Gehäusemantel kann über Lufteinlässe (nicht dargestellt) seiner Unterseite den Luftstrom A aus dem Bereich des Kochfelds aufnehmen und mittels eines entsprechenden Lüfters der Dunstabzugshaube durch sich hindurchbefördern. Im Bereich des oberen Gehäusemantels ist eine entsprechende Filterung im Inneren vorgesehen, welche Gerüche sowie Fette aus dem Luftstrom A herausfiltern kann. Der gefilterte Luftstrom A kann die Dunstabzugshaube über dessen Oberseite und dort vorgesehene Luftauslässe wieder verlassen. Um die Luftauslässe des oberen Gehäusemantels herum verteilt werden die unteren Enden der vier Seile aufgenommen, so dass die eigentliche Dunstabzugshaube von den Seilen sowie der Deckenhalterung an einer Decke gehalten werden kann.

Es kann zusätzlich eine Aufnahme für den Duftstoffquellenhalter 1 einer Beduftungseinheit in der Oberseite des oberen Gehäusemantels vorgesehen sein. In der Aufnahme wird der Duftstoffquellenhalter 1 der Beduftungseinheit 1 aufgenommen. Die Funktion der Beduftungseinheit bzw. des Duftstoffquellenhalter 1 wurde zuvor bereits beschrieben.

Die Beduftungseinheit 1 kann dabei über einen, zumindest teilweise separaten Luftkanlal verfügen und einen eigenen elektrischen Lüfter aufweisen, welcher unabhängig von dem Lüfter der Dunstabzugshaube betrieben werden kann.

### Bezugszeichenliste (Bestandteil der Beschreibung)

- A: Luftstrom bzw. dessen Strömungsrichtung
- X: Längsrichtung; Tiefe
- Y: Querrichtung; Breite
- Z: vertikale Richtung; Höhe

- 1: Duftstoffquellenhalter; Duftstoffbehälterhalter; Duftstoffkapselhalter; Duftflaconhalter
- 10: Oberteil
- 10a: Verbindungselemente; Rasthaken
- 10b: Führungselement; Vorsprung
- 11: Unterteil
- 11a: Verbindungselementaufnahmen; Rasthakenaufnahmen
- 12: Aufnahme der Duftstoffquelle
- 12a: Aufnahme für Verbindungselement der Duftstoffquelle
- 12b: Codierungselement für Duftstoffquelle; Aussparung
- 13: Oberseite; Außenseite
- 14: Auslassöffnungen; Auslassschlitze
- 15: Betätigungselement, Fingermulde
- 16: Auffangelement; Auffangbehälter; Auffangwanne
- 17: Abstellelement; (hohle) Standfüße
- 18: Verbindungselement für Halterungseinheit; Rastelement bzw. Rasthaken für Push-Push-Verriegelungsmechanismus
- 19: Aufnahme für Führungselement; Nut für Führungszapfen

## Patentansprüche

1. Haushaltsgerät mit einem Duftstoffquellenhalter (1) und mit einer Beduftungseinheit zur Aufnahme eines Duftstoffquellenhalters (1),
wobei der Duftstoffquellenhalter (1) ausgebildet ist, aus der Beduftungseinheit entnommen und in die Beduftungseinheit eingeführt zu werden,
wobei der Duftstoffquellenhalter (1) wenigstens eine Aufnahme (12) für wenigstens eine Duftstoffquelle aufweist, wobei die Aufnahme (12) horizontal ausgerichtet ist,
wobei der Duftstoffquellenhalter (1) wenigstens ein Auffangelement (16) aufweist, wobei das Auffangelement (16) in der Höhe (Z) unterhalb der Aufnahme (12) angeordnet ist, welche für den Fall eines Austritts eines flüssigen Duftstoffs aus der Duftstoffquelle zum Auffangen des flüssigen Duftstoffs vorgesehen ist,
wobei der Duftstoffquellenhalter (1) wenigstens ein Abstellelement (17) aufweist, welches ausgebildet ist, dem aus der Beduftungseinheit entnommenen Duftstoffquellenhalter (1) einen sicheren Stand zu bieten, in welchem die Aufnahme (12) horizontal ausgerichtet ist,
**dadurch gekennzeichnet, dass**
das Haushaltsgerät als Dunstabzugshaube ausgeführt ist und
die Beduftungseinheit mit einem Lüfter zur Erzeugung eines Luftstroms ausgebildet ist, wobei die Beduftungseinheit von dem Haushaltsgerät elektrisch Versorgungt wird.

2. Haushaltsgerät nach dem vorangehenden Anspruch, **wobei** das Volumen des Auffangelements (16) ist gleich groß oder größer als der Inhalt einer einzelnen Duftstoffquelle dimensioniert und somit ausreichend groß ausgebildet ist, um die gesamte aus der Duftstoffquelle austretende Duftstoffflüssigkeit aufzunehmen.

3. Haushaltsgerät nach einem der vorangehenden Ansprüche, **wobei** das Abstellelement (17) als mehrere Standfüße (17) ausgebildet ist.

4. Haushaltsgerät nach dem vorangehenden Anspruch, **wobei** das Abstellelement (17) als vier Standfüße (17) ausgebildet ist, welche vorzugsweise in den Ecken des Duftstoffquellenhalters (1) angeordnet sind.

5. Haushaltsgerät nach einem der beiden vorangehenden Ansprüche, **wobei** zwischen den Standfüßen (17) wenigstens ein Verbindungselement (18), vorzugsweise ein Rastelement (18), besonders vorzugsweise ein Rasthaken (18), für eine Halterungseinheit, angeordnet ist, wobei das Verriegelungselement (18) in der Höhe (Z) gegenüber den Standfüßen (17) gleich lang oder zurückversetzt ausgebildet ist.

6. Haushaltsgerät nach einem der beiden vorangehenden Ansprüche, **wobei** die Halterungseinheit mit dem Verbindungselement (18) als ein Push-Push-Verriegelungsmechanismus ausgeführt ist.

7. Haushaltsgerät nach einem vorangehenden Ansprüche, **wobei** das Auffangelement (16) als Abstellelement (17) ausgebildet ist.

8. Haushaltsgerät nach einem der vorangehenden Ansprüche, **wobei**
der Duftstoffquellenhalter (1) wenigstens zweiteilig ausgebildet ist und wenigstens ein Oberteil (10) und wenigstens ein Unterteil (11) aufweist,
wobei das Oberteil (10) wenigstens die Aufnahme (12) aufweist und
wobei das Unterteil (11) wenigstens ein Auffangelement (16) und/oder wenigstens ein Abstellelement (17) aufweist.

9. Haushaltsgerät nach dem vorangehenden Anspruch, **wobei**
das Oberteil (10) wenigstens ein Verbindungselement (10a), vorzugsweise eine Mehrzahl von Verbindungselementen (10a), und das Unterteil (11) wenigstens eine Verbindungselementaufnahme (11a), vorzugsweise eine Mehrzahl von Verbindungselementaufnahmen (11a), aufweist, oder umgekehrt,
wobei vorzugsweise das Verbindungselement (10a) als Rasthaken (10a) und die Verbindungselementaufnahme (11a) als Rasthakenaufnahme (11a) ausgebildet ist.

## Claims

1. Domestic appliance comprising a fragrance-source holder (1) and comprising a fragrancing unit for receiving a fragrance-source holder (1),
wherein the fragrance-source holder (1) is designed to be removed from the fragrancing unit and inserted into the fragrancing unit,
wherein the fragrance-source holder (1) comprises at least one receptacle (12) for at least one fragrance source, wherein the receptacle (12) is horizontally oriented,
wherein the fragrance-source holder (1) comprises at least one collecting element (16), wherein the collecting element (16) is arranged vertically (Z) below the receptacle (12) which is provided for collecting the liquid fragrance in the event of a liquid fragrance escaping from the fragrance source,
wherein the fragrance-source holder (1) comprises at least one stand element (17) which is designed to provide a secure stand for the fragrance-source holder (1) removed from the fragrancing unit, in which stand the receptacle (12) is horizontally oriented,
**characterised in that**
the domestic appliance is designed as an extractor hood and
the fragrancing unit has a fan for generating an air flow, wherein the fragrancing unit is electrically supplied by the domestic appliance.

2. Domestic appliance according to the preceding claim, **wherein** the volume of the collecting element (16) is designed to be equal to or larger than the content of an individual fragrance source and is thus large enough to receive all of the fragrance liquid escaping from the fragrance source.

3. Domestic appliance according to either of the preceding claims, **wherein** the stand element (17) is in the form of a plurality of feet (17).

4. Domestic appliance according to the preceding claim, **wherein** the stand element (17) is in the form of four feet (17) which are preferably arranged at the corners of the fragrance-source holder (1).

5. Domestic appliance according to either of the two preceding claims, **wherein** at least one connection element (18), preferably a catch element (18), particularly preferably a catch hook (18), for a holding unit is arranged between the feet (17), wherein the locking element (18) is designed to be the same length as or set back vertically (Z) with respect to the feet (17).

6. Domestic appliance according to either of the two preceding claims, **wherein** the holding unit having the connection element (18) is designed as a push-push locking mechanism.

7. Domestic appliance according to any of the preceding claims, **wherein** the collecting element (16) is in the form of a stand element (17).

8. Domestic appliance according to any of the preceding claims, **wherein**
the fragrance-source holder (1) is formed in at least two parts and comprises at least one upper part (10) and at least one lower part (11),
wherein the upper part (10) comprises at least the receptacle (12) and
wherein the lower part (11) comprises at least one collecting element (16) and/or at least one stand element (17).

9. Domestic appliance according to the preceding claim, **wherein**
the upper part (10) comprises at least one connection element (10a), preferably a plurality of connection elements (10a), and the lower part (11) comprises at least one connection element receptacle (11a), preferably a plurality of connection element receptacles (11a), or vice versa,
wherein preferably the connection element (10a) is in the form of a catch hook (10a) and the connection element receptacle (11a) is in the form of a catch-hook receptacle (11a).

## Revendications

1. Appareil électroménager comportant un support de source de parfum (1) et comportant une unité de diffusion de parfum permettant la réception d'un support de source de parfum (1),
dans lequel le support de source de parfum (1) est réalisé pour être retiré de l'unité de diffusion de parfum et pour être introduit dans l'unité de diffusion de parfum,
dans lequel le support de source de parfum (1) présente au moins un réceptacle (12) pour au moins une source de parfum, dans lequel le réceptacle (12) est orienté horizontalement,
dans lequel le support de source de parfum (1) présente au moins un élément de recueil (16), dans lequel l'élément de recueil (16) est disposé à la hauteur (Z) en dessous du réceptacle (12), lequel élément de recueil est prévu pour le recueil du parfum liquide en cas d'un écoulement d'un parfum liquide depuis la source de parfum,
dans lequel le support de source de parfum (1) présente au moins un élément de pose (17) qui est réalisé pour offrir une base sûre au support de source de parfum (1) retiré de l'unité de diffusion de parfum, dans lequel le réceptacle (12) est orienté horizontalement,
**caractérisé en ce que**
l'appareil électroménager est conçu sous forme de hotte aspirante et
l'unité de diffusion de parfum est réalisée avec un ventilateur pour la génération d'un flux d'air, dans lequel l'unité de diffusion de parfum est alimentée électriquement par l'appareil électroménager.

2. Appareil électroménager selon la revendication précédente, **dans lequel** le volume de l'élément de recueil (16) est dimensionné de manière à être égal ou supérieur au contenu d'une seule source de parfum et est donc réalisé de manière suffisamment grande pour recevoir la totalité du liquide de parfum s'écoulant depuis la source de parfum.

3. Appareil électroménager selon l'une des revendications précédentes, **dans lequel** l'élément de pose (17) est réalisé sous forme de plusieurs pieds de base (17).

4. Appareil électroménager selon la revendication précédente, **dans lequel** l'élément de pose (17) est réalisé sous forme de quatre pieds de base (17) qui sont de préférence disposés dans les coins du support de source de parfum (1).

5. Appareil électroménager selon l'une des deux revendications précédentes, **dans lequel** au moins un élément de liaison (18), de préférence un élément d'encliquetage (18), de manière particulièrement préférée un crochet d'encliquetage (18), pour une unité de support, est disposé entre les pieds de base (17), dans lequel l'élément de verrouillage (18) est réalisé avec la même longueur ou en retrait à la hauteur (Z) par rapport aux pieds de base (17).

6. Appareil électroménager selon l'une des deux revendications précédentes, **dans lequel** l'unité de support comportant l'élément de liaison (18) est conçue sous forme de mécanisme de verrouillage push-push.

7. Appareil électroménager selon l'une des revendications précédentes, **dans lequel** l'élément de recueil (16) est réalisé sous forme d'élément de pose (17).

8. Appareil électroménager selon l'une des revendications précédentes, **dans lequel**
le support de source de parfum (1) est réalisé au moins en deux parties et présente au moins une partie supérieure (10) et au moins une partie inférieure (11),
dans lequel la partie supérieure (10) présente au moins le réceptacle (12) et
dans lequel la partie inférieure (11) présente au moins un élément de recueil (16) et/ou au moins un élément de pose (17).

9. Appareil électroménager selon la revendication précédente, **dans lequel**
la partie supérieure (10) présente au moins un élément de liaison (10a), de préférence une pluralité d'éléments de liaison (10a), et la partie inférieure (11) présente au moins un réceptacle d'élément de liaison (11a), de préférence une pluralité de réceptacles d'élément de liaison (11a), ou inversement,
dans lequel l'élément de liaison (10a) est de préférence réalisé sous forme de crochet d'encliquetage (10a) et le réceptacle d'élément de liaison (11a) est réalisé sous forme de réceptacle de crochet d'encliquetage (11a).
